# EUROPÄISCHE PATENTANMELDUNG

(11) **EP 4 663 095 A1**
(43) Veröffentlichungstag der Anmeldung: **17.12.2025**
(21) Anmeldenummer: 24182257.6
(22) Anmeldetag: 14.06.2024
(51) Int. Cl.: A61B 1/12, A61B 1/233, A61B 1/24, A61B 1/267

(54) **SAUGVORRICHTUNG UND INTUBATIONSGERÄT**

(71) Anmelder: Medizinische Hochschule Hannover, 30625 Hannover (DE); Hebei Vimed Medical Device Co, Ltd, Langfang, Hebei 065201 (CN)
(72) Erfinder: RAYMONDOS, Konstantinos, 30655 Hannover (DE); CHI, Liguang, LangFang, Hebei Province, 065201 (CN)
(74) Vertreter: Günther, Constantin

(57) **Zusammenfassung**

Die Erfindung betrifft eine Saugvorrichtung, insbesondere eine medizinische Saugvorrichtung, eingerichtet zur Anbringung an einem Intubationsgerät zum Intubieren eines Patienten mit einem Endotrachealtubus, wobei das Intubationsgerät einen Handgriff zum Halten des Intubationsgeräts und eine mit dem Handgriff verbundene starre Führungsschiene hat, die einen in Längsrichtung von einem patientenfernen Ende bis zu einem patientennahen Ende der Führungsschiene verlaufenden Führungskanal zum Führen des Endotrachealtubus und wenigstens eine optische Erfassungseinrichtung am patientennahen Ende an der Führungsschiene hat, wobei die Saugvorrichtung zum Absaugen von Flüssigkeiten im Bereich des patientennahen Endes der Führungsschiene eingerichtet ist. Die Erfindung betrifft außerdem ein Intubationsgerät zum Intubieren eines Patienten mit einem Endotrachealtubus, die eine Saugvorrichtung aufweist.

## Beschreibung

Die Erfindung betrifft eine Saugvorrichtung, insbesondere eine medizinische Saugvorrichtung, eingerichtet zur Anbringung an einem Intubationsgerät zum Intubieren eines Patienten mit einem Endotrachealtubus, wobei das Intubationsgerät einen Handgriff zum Halten des Intubationsgeräts und eine mit dem Handgriff verbundene starre Führungsschiene hat, die einen in Längsrichtung von einem patientenfernen Ende bis zu einem patientennahen Ende der Führungsschiene verlaufenden Führungskanal zum Führen des Endotrachealtubus und wenigstens eine optische Erfassungseinrichtung am patientennahen Ende an der Führungsschiene hat, wobei die Saugvorrichtung zum Absaugen von Flüssigkeiten im Bereich des patientennahen Endes der Führungsschiene eingerichtet ist. Die Erfindung betrifft außerdem ein Intubationsgerät zum Intubieren eines Patienten mit einem Endotrachealtubus, die eine Saugvorrichtung aufweist.

### Einleitung

In einer medizinischen Endoskopie ist die Vermeidung einer Sichtblockade durch Flüssigkeiten und die Reinigung der Endoskopoptik durch Absaugen und Spülung Standard. Das Absaugen und die Spülung erfolgt meistens über einen Arbeitskanal des Endoskops. Darüber hinaus können Endoskope oder Videolaryngoskope auch mit einer Absaugvorrichtung versehen werden. Allerdings kann trotz Absaugung die endoskopische Sicht durch Flüssigkeiten blockiert werden, so dass eine mechanische Reinigung erfolgen muss. Dies kann durch eine Spülung oder durch Entfernen des Endoskops und dann erfolgender Reinigung erfolgen.

### Stand der Technik

### Endoskopie

Bei den jetzigen Standard - Endoskopen erfolgt die Spülung und die Absaugung durch einen Arbeitskanal, der bereits in das Endoskop integriert ist. Kombinationen aus Mehrweg-Endoskopen und Einweg - Arbeits- bzw. Absaugkanälen wurden bereits vor Jahrzehnten eingeführt. Hierdurch entfällt die Reinigung und Aufbereitung des sehr dünnen Arbeitskanals. In letzter Zeit wird das Problem zunehmend dadurch gelöst, in dem Endoskope mit integriertem Arbeitskanal als Einwegprodukt vertrieben werden.

### Videolaryngoskopie

Im Gegensatz zu Endoskopen oder Videotuben gibt es bei Videolaryngoskopen keinen integrierten Kanal, über den abgesaugt und/oder die Optik gespült werden kann. Es gibt nur Kanäle, die in die Nähe der Optik führen und über die ein Standardabsaugschlauch eingeführt werden kann. Beispielsweise können Absaugkatheter seitlich oder innerhalb des Videolaryngskopiespatels geführt werden, um eine Absaugung und somit eine sichere Sicht während der Laryngoskopie zu ermöglichen.

Alle diese Methoden sind allerdings so ineffizient und unsicher, dass sie in der klinischen Routine praktisch nicht verwendet werden. Außer den beiden genannten Geräten gibt es auch keine weiteren Videolaryngoskope, bei denen eine Absaugvorrichtung integriert werden kann.

Vielmehr wird in der Regel ein vom Videolaryngoskop unabhängiger rigider Sauger mit einer speziellen Krümmung durch eine zweite Person eingeführt - z.B. ein sog. Yankauer - oder Tonsillen - Sauger. Hiermit wird versucht, zum tiefsten Punkt im Bereich der oberen Atemwege zu gelangen: Hier sammeln sich bei liegenden Patienten regurgitierter Mageninhalt, Sekret oder Blut aus Mund, Nase oder Rachen an und gelangen dann zum Kehlkopfeingang. Diese Flüssigkeiten versperren dann die Sicht, was eine erfolgreiche Atemwegssicherung erschweren oder sogar unmöglich machen kann. Darüber hinaus kann der Eintritt von Magensäure in die Lunge zu einem ausgeprägtem Lungenversagen führen (ARDS), was tödlich verlaufen kann.

Aus der EP 4 218 534 A1 ist ein kombiniertes Saug-Spül-System für ein medizinisches Instrument bekannt.

### Probleme und Nachteile beim bisherigen Stand der Technik

### Endoskopie

Bei der klassischen Endoskopie in der Lunge oder im Gastrointestinaltrakt ist der im Endoskop integrierte Arbeitskanal schwierig aufzubereiten, entsprechende Einwegendoskope sind pro Anwendung sehr teuer.

Bei starren Endoskopen wird wie bei der Videolaryngoskopie bei Bedarf in der Regel zusätzlich ein starrer Sauger eingeführt.

### Videolaryngoskopie

Sowohl das Absaugen über an Spateln befestigte Absaugkatheter als auch das häufiger praktizierte Absaugen über einen zusätzlich zum Videolaryngoskop eingeführten Sauger weisen erhebliche Probleme und Nachteile auf:

### Nachteile der an Spateln befestigten Absaugschläuche

Eine sichere und gezielte Positionierung der Absaugöffnung ist nicht oder nur sehr schwer möglich, da der flexible Schlauch abknicken und zudem nicht sicher gesteuert werden kann:
1. Der flexible Absaugschlauch knickt leicht beim Einführen des Videolaryngoskopiespatels ab. Bei abgeknicktem Schlauch ist keine Absaugung mehr möglich, da sich der Sog nicht bis zum Schlauchende fortsetzten kann.
2. Bei erhaltener Öffnung des Schlauchlumens setzt sich der Sog zwar bis zur Spitze des Schlauches fort. Trotzdem biegt sich der flexible Schlauch in den engen oberen Atemwegen und das Schlauchende ist nicht führbar.
3. Darüber hinaus kann das Schlauchende nicht unabhängig von der Spatelposition gesteuert werden. Wenn also der Spatel angehoben wird, um eine optimale Sicht für die Intubation zu erreichen, wird auch der flexible Absaugschlauch bewegt. Er kann somit dann auch nicht gezielt, verlässlich und damit sicher auf einen gewünschten Punkt - wie z.B. dem tiefsten Punkt unter dem Kehlkopf direkt vor der Speiseröhre - gerichtet werden.
4. Wird ein Absaugschlauch durch den Tubus geführt, so ist die Positionierung zusätzlich noch von der Position des Tubus abhängig: So kann nicht gleichzeitig unterhalb des Kehlkopfes abgesaugt und weiter oben in den Kehlkopf hinein intubiert werden. Dies kann zur Folge haben, dass die Sicht durch Flüssigkeit versperrt wird, sobald der Sauger zusammen mit dem Tubus nach oben in den Kehlkopfeingang gerichtet wird.
5. Der Nachteil aller bisherigen Möglichkeiten ist, dass die Aktivierung der Absaugung durch Verschluss des sogenannten Fingertip - Konnektors erfolgen muss. Das ist umständlich bzw. muss durch eine zweite Person erfolgen, da nicht gleichzeitig das Laryngoskop gehalten, der Tubus vorgeführt und die Saugung aktiviert werden kann, wenn der Fingertip nicht am Laryngoskop an leicht zugänglicher Stelle befestigt wird. Mit der gerätesteuernden Hand kann also nicht gleichzeitig abgesaugt und das Gerät gelenkt werden, so dass die zweite Hand nicht für eine Intubation zur Verfügung steht. Ist der Fingertip - Konnektor dauerhaft verschlossen, wird kontinuierlich gesaugt, was rasch zu einem Ansaugen an den Schleimhäuten führt.
6. Ein weiterer Nachteil der fehlenden Führung des Absaugschlauches ist, dass sich das Schlauchende leicht an der Schleimhaut festsaugen kann und somit erneut - wie oben unter 1. beschrieben- der Sog verloren geht und Flüssigkeit nicht mehr abgesaugt werden kann: Das ist auch schnell bei zusätzlichen, seitlichen Schlauchöffnungen der Fall, die eigentlich ein Ansaugen verhindern sollen. Ausbuchtungen und Taschen im Bereich der oberen Atemwege können leicht das komplette Schlauchende mit allen Öffnungen umhüllen.

Wegen dieser erheblichen Nachteile werden wie oben bereits erwähnt, im Videolaryngoskop integrierte Absaugschläuche praktisch nicht verwendet, sondern vom Laryngoskop unabhängige, rigide und gekrümmte Sauger benutzt:

### Nachteile eines zusätzlich eingeführten, rigiden Saugers

In der Regel wird der bereits vorbereitete und unter kontinuierlichem Sog stehende Sauger erst dann eingeführt, wenn sich Flüssigkeit vor dem Kehlkopfeingang ansammelt und dann die Sicht blockiert. Das wird so praktiziert, weil das gleichzeitige Einführen von Spatel, Sauger und Tubus in dem engen Bereich der oberen Atemwege ebenfalls erhebliche Probleme aufweist:
7. Bei eingeführtem Spatel und Sauger kann durch die räumliche Enge eine gezielte Tubusführung zum Kehlkopfeingang bei Videolaryngoskopen mit freier Tubusführung erheblich erschwert oder sogar unmöglich werden: Die laryngoskopierende Person kann nicht gezielt saugen und gleichzeitig intubieren - nur nacheinander. Soll während der Intubation gleichzeitig gesaugt werden, muss der Sauger für eine erfolgreiche Atemwegssicherung von einer zweiten Person an den äußersten rechten Mundwinkel gehalten werden, um den erforderlichen Platz für eine Intubation zu schaffen. Gleichzeitig wird dabei versucht, die Saugerspitze an den tiefsten Punkt zu positionieren und hier zu halten, ohne die Intubation zu behindern.
8. Zwar ist ein gezieltes Saugen und gleichzeitiges Intubieren wie oben unter 7. beschrieben ohne eine weitere Person nicht möglich. Allerdings kann eine Person ungezielt saugen, wobei sowohl der rigide Sauger als auch das Videolaryngoskop mit der linken Hand umfasst bzw. gehalten werden können. Durch die Positionierung des Saugers auf der linken Seite, wird die Behinderung der Tubusführung auf der rechten Seite des Videolaryngoskops sicher vermieden.

Der rigide Sauger kann beispielsweise links ohne optische Führung in den Rachen positioniert werden. Das Videolaryngoskop wird danach oder vorher mit der linken Hand eingeführt. Die linke Hand kann dann sowohl das Videolaryngoskop als auch den Sauger umfassen, so dass dann mit der rechten Hand intubiert werden kann.

Allerdings kann hierbei die Spitze des Saugers sehr schwer oder nicht gesteuert werden, so dass sich die Saugeröffnung wie oben unter 6. beschrieben leicht ansaugen kann. Dadurch kann der Sog vollständig verloren gehen und Flüssigkeit kann dann nicht mehr abgesaugt werden.

### Nachteile einer fehlenden Kontrolle über Effizienz und Funktionsfähigkeit der Saugung

9. Ob und in welchem Ausmaß überhaupt eine effiziente Saugung besteht, kann in der Regel gar nicht sicher während der Laryngoskopie festgestellt werden. Die hierfür erforderliche Druckanzeige ist direkt an der Vakuumquelle angebracht. Diese Quelle befindet sich im unteren Bereich des Narkosegeräts und liegt somit außerhalb der Sichtweite.

### Zusammenfassung der Probleme beim bisherigen Stand der Technik

Zusätzlich zu den fünf Problembereichen, die bereits in der EP 4 218 534 A1 beschrieben wurden, gibt es weitere neun Probleme die eine effektive und sichere Absaugung während der Videolaryngoskopie erheblich erschweren oder sogar verhindern:
1. Das Abknicken des flexiblen Absaugschlauch unterbricht den Sog.
2. Die Flexibilität des Schlauches erschwert oder verhindert eine sichere Führung.
3. Der Schlauch bewegt sich mit dem Spatel: Die Positionierung hängt also von der für die Öffnung der Atemwege notwendigen Spatelposition ab - eine von dieser Spatelbewegung unabhängige Steuerung der Absaugung ist nicht möglich.
4. Bei einem Absaugschlauch im Tubus hängt die Führung zusätzlich noch von der Tubusposition ab.
5. Die Aktivierung des Sogs ist umständlich und erschwert, so dass Hilfe durch eine zweite Person erforderlich wird.
6. Durch die mangelnde Führung saugt sich der Absaugschlauch an der Schleimhaut leicht fest, was zu einer Verminderung oder zu einem Verlust des Sogs führt.
7. Durch einen rechts eingeführten, rigiden Sauger wird die Intubation erschwert. Das Halten der optimalen Saugerposition und ein Verhindern von Ansaugen ist ebenfalls schwierig.
8. Bei einen links eingeführtem, rigiden Sauger ist die Steuerung unzureichend, wodurch ein hohes Risiko des Ansaugens besteht - mit nachfolgender Verminderung oder vollständigem Verlust des Sogs.
9. Während der Laryngoskopie kann die Effizienz und die Funktionsfähigkeit der Saugung nicht verlässlich und sicher kontrolliert werden.

Der Erfindung liegt die Aufgabe zugrunde, unter Vermeidung der zuvor erläuterten Nachteile verbesserte Absaugmöglichkeiten bei medizinischen Eingriffen, insbesondere bei der Intubation, anzugeben.

Diese Aufgabe wird mit einer Saugvorrichtung der eingangs genannten Art gelöst, indem die Saugvorrichtung wenigstens eine Ansaugöffnung zum Ansaugen der Flüssigkeiten hat, die relativ zu einer beim Intubationsvorgang der Zunge des Patienten zugewandten Oberseite der Führungsschiene an einer Position unterhalb der wenigstens einen optischen Erfassungsvorrichtung oder unterhalb der Führungsschiene angeordnet ist, wenn Saugvorrichtung am Intubationsgerät befestigt ist. Relativ zur Oberseite der Führungsschiene ist die wenigstens eine Ansaugöffnung somit tiefer als die optische Erfassungsvorrichtung bzw. benachbarte Bereiche der Führungsschiene gelegen, sodass die Absaugung am tiefstmöglichen Punkt erfolgen kann. Dementsprechend können auftretende Flüssigkeiten durch die Saugvorrichtung bereits abgesaugt werden, bevor sie die optische Erfassungsvorrichtung erreichen und dadurch die Sicht beeinträchtigen. Damit ist die optische Erfassungseinrichtung gut vor Verschmutzung geschützt.

Die optische Erfassungseinrichtung kann z.B. durch eine am patientennahen Ende der Führungsschiene angeordnete Kamera, eine Linse oder ein Objektiv gebildet sein. Wird zur optischen Erfassung ein durch die Führungsschiene verlegter Lichtleiter verwendet, so wird die am patientennahen Ende der Führungsschiene angeordnete Endseite des Lichtleiters als optische Erfassungseinrichtung angesehen.

Die erfindungsgemäße Saugvorrichtung kann als Zusatzkomponente ausgebildet sein, die an einem Intubationsgerät befestigt werden kann. Die Anbringung der Saugvorrichtung am Intubationsgerät ist dabei optional und kann je nach Anwendungsfall durchgeführt werden oder entfallen. Die erfindungsgemäße Saugvorrichtung kann auch in ein medizinisches Instrument integriert ausgebildet sein, z.B. in einem Intubationsgerät, wie nachfolgend noch erläutert wird.

Gemäß einer vorteilhaften Ausgestaltung der Erfindung ist vorgesehen, dass die wenigstens eine Ansaugöffnung weiter vom patientenfernen Ende entfernt ist als die wenigstens eine optische Erfassungsvorrichtung. Auf diese Weise ist die Ansaugposition relativ weit nach vorn verlagert, insbesondere vor der optischen Erfassungsvorrichtung. Auch hierdurch kann die Effizienz der Absaugung weiter verbessert werden, wobei die optische Erfassungseinrichtung noch besser vor Verschmutzungen geschützt werden kann, indem Flüssigkeiten vor Erreichen der optischen Erfassungseinrichtung zuverlässig abgesaugt werden können.

Gemäß einer vorteilhaften Ausgestaltung der Erfindung ist vorgesehen, dass die Saugvorrichtung einen Hauptkörper hat, der über seine gesamte Längserstreckung oder zumindest den wesentlichen Teil seiner Längserstreckung einem in Längsrichtung gekrümmten Verlauf der Führungsschiene folgt. Als Längserstreckung des Hauptkörpers wird dabei die Richtung angesehen, in der der Hauptkörper seine größte Dimension hat. Hierdurch kann die Saugvorrichtung mit ihrem Hauptkörper besonders effizient mit dem Intubationsgerät und deren Führungsschiene zu einer Baueinheit verbunden werden, die kaum größer ist als die Führungsschiene selbst. Die durch die an der Führungsschiene angebrachte Saugvorrichtung resultierenden Abmessungen sind nur minimal größer als ohne die Saugvorrichtung. Auf diese Weise wird der Intubationsvorgang durch die Saugvorrichtung nicht zusätzlich erschwert. Insbesondere sind weiterhin auch einfache und zügige Intubationen bei schwierigen Atemwegen möglich.

Gemäß einer vorteilhaften Ausgestaltung der Erfindung ist vorgesehen, dass der Hauptkörper wenigstens ein patientennahes Befestigungselement hat, mit dem der Hauptkörper an einer ersten Befestigungsstelle am patientennahen Ende der Führungsschiene anrastbar ist, und der Hauptkörper wenigstens ein patientenfernes Befestigungselement hat, mit dem der Hauptkörper an einer zweiten Befestigungsstelle am patientenfernen Ende der Führungsschiene und/oder dem Handgriff befestigbar ist. Dies erlaubt ein schnelles und intuitives Befestigen der Saugvorrichtung am Intubationsgerät. Die Befestigung der Saugvorrichtung am Intubationsgerät ist damit auch in Notfall- und Stresssituationen zuverlässig möglich. Durch ein einfaches Anrasten des Hauptkörpers an der Führungsschiene ist die Befestigung auch wieder leicht lösbar.

Gemäß einer vorteilhaften Ausgestaltung der Erfindung ist vorgesehen, dass der Hauptkörper unter Spannung zwischen der ersten und der zweiten Befestigungsstelle einspannbar ist. Dementsprechend ist der Hauptkörper an gegenüberliegenden Enden am Intubationsgerät festgespannt. Der Hauptkörper ist damit in seiner Längserstreckungsrichtung mit einer gewissen, relativ geringen mechanischen Zugspannung belastet.

Gemäß einer vorteilhaften Ausgestaltung der Erfindung ist vorgesehen, dass das patientennahe Befestigungselement als eine in Richtung zum patientenfernen Ende abragende Rastkante ausgebildet ist. Das patientennahe Befestigungselement kann z.B. einstückig an das patientennahe Ende des Hauptkörpers angeformt sein. Beispielsweise kann der Hauptkörper am patientennahen Ende einen gekrümmten Wulst haben, der an die Außenkontur der Führungsschiene am patientennahen Ende angepasst ist. Das patientennahe Befestigungselement kann als Vorsprung ausgebildet sein, der von diesem Wulst etwas in Richtung zum patientenfernen Ende abragt.

Gemäß einer vorteilhaften Ausgestaltung der Erfindung ist vorgesehen, dass der Hauptkörper als im Wesentlichen starres, schienenartiges Bauteil ausgebildet ist. Dies ist vorteilhaft für ein sicheres und einfaches Hantieren mit der Saugvorrichtung, z.B. beim Befestigen am Intubationsgerät oder beim Lösen vom Intubationsgerät. Durch die starre, schienenartige Ausführung können zudem in dem Hauptkörper verlaufende Saugkanäle gestützt werden und vor unerwünschter Kompression oder einem Abknicken geschützt werden. Der Hauptkörper kann z.B. als Saugschiene ausgebildet sein.

Gemäß einer vorteilhaften Ausgestaltung der Erfindung ist vorgesehen, dass der Hauptkörper einen ersten Seitenabschnitt einen davon beanstandeten zweiten Seitenabschnitt hat, wobei der erste und der zweite Seitenabschnitt im Wesentlichen parallel zueinander in Längsrichtung entlang der Führungsschiene verlaufen, wenn Saugvorrichtung am Intubationsgerät befestigt ist, wobei die Führungsschiene zwischen dem ersten und dem zweiten Seitenabschnitt aufnehmbar ist. Dies hat den Vorteil, dass der Hauptkörper die Führungsschiene an beiden Seiten umgreifen kann, sodass der Hauptkörper besonders sicher an der Führungsschiene befestigt werden kann und im daran befestigten Zustand durch die Führungsschiene zusätzlich stabilisiert ist, insbesondere in seitlicher Richtung. Der Hauptkörper muss dadurch nicht übermäßig stabil sein, insbesondere nicht so stabil wie die Führungsschiene. Beispielsweise kann der Hauptkörper als leichtes und kostengünstiges Kunststoffbauteil bereitgestellt werden. Der erste und der zweite Seitenabschnitt müssen nicht über ihre gesamte Längserstreckung parallel zueinander verlaufen. Insbesondere im Bereich des Handgriffs können die Seitenabschnitte nicht genau parallel verlaufen, sondern nach außen hin ausgebuchtet sein, wie nachfolgend erläutert wird.

Gemäß einer vorteilhaften Ausgestaltung der Erfindung ist vorgesehen, dass der erste und der zweite Seitenabschnitt im Bereich des Handgriffs jeweils mit einem Abstand zum Handgriff an diesem vorbeigeführt sind, sodass der erste und der zweite Seitenabschnitt den Handgriff seitlich nicht berühren. Auf diese Weise wird an dem Hauptkörper im Bereich des Handgriffs eine gewisse Ausbuchtung nach außen realisiert, sodass die Seitenabschnitte nicht direkt am Hauptkörper anliegen. Auch hierdurch kann eine unerwünschte Kompression der im Hauptkörper verlegten Saugkanäle vermieden werden.

Der Hauptkörper kann eine im Wesentlichen offene Form haben, indem zwischen dem ersten und dem zweiten Seitenabschnitt über die überwiegende Längserstreckung keine Verbindung vorhanden ist. Beispielsweise kann der erste und der zweite Seitenabschnitt lediglich am patientenfernen Ende durch einen Quersteg und zusätzlich am patientennahen Ende durch einen weiteren Quersteg miteinander verbunden sein.

Gemäß einer vorteilhaften Ausgestaltung der Erfindung ist vorgesehen, dass der erste und der zweite Seitenabschnitt durch einen Bodenabschnitt miteinander verbunden sind. Auf diese Weise können der erste und der zweite Seitenabschnitt auch im mittleren Bereich, d.h. im Bereich zwischen dem patientennahen und dem patientenfernen Ende, miteinander durch den Bodenabschnitt verbunden sein. Hierdurch kann der Hauptkörper mit geringem Aufwand noch stabiler gestaltet werden. Dabei muss sich der Bodenabschnitt nicht unbedingt vollständig vom patientennahen Ende bis zum patientenfernen Ende erstrecken, sondern kann beispielsweise nur als ein relativ kurzer Bodenabschnitt im mittleren Bereich oder als mehrere durch Aussparungen voneinander getrennte Bodenabschnitt-Stücke ausgebildet sein.

Gemäß einer vorteilhaften Ausgestaltung der Erfindung ist vorgesehen, dass der Bodenabschnitt im am Intubationsgerät befestigten Zustand der Saugvorrichtung unterhalb der Unterseite der Führungsschiene angeordnet ist. Auf diese Weise ist der Hauptkörper zusätzlich in einer weiteren Raumrichtung zuverlässig an der Führungsschiene fixierbar. Zudem wird vermieden, dass durch den Hauptkörper der Führungskanal der Führungsschiene und damit das Hindurchführen des Endotrachealtubus (nachfolgend auch kurz "Tubus" genannt) beeinträchtigt wird.

In einer vorteilhaften Ausgestaltung kann die Saugvorrichtung keine Bauteile haben, die im Bereich des Führungskanals der Führungsschiene angeordnet sind. Insbesondere kann die Saugvorrichtung ohne Bauteile ausgebildet sein, die sich an der Oberseite der Führungsschiene befinden. Die Saugvorrichtung hat in diesem Fall somit nur Bauteile, die sich links und rechts seitlich der Führungsschiene sowie unterhalb der Führungsschiene befinden können.

Gemäß einer vorteilhaften Ausgestaltung der Erfindung ist vorgesehen, dass der Hauptkörper wenigstens eine Montageöffnung hat, durch die der Handgriff des Intubationsgeräts beim Vorgang des Befestigens der Saugvorrichtung am Intubationsgerät zwischen dem ersten und dem zweiten Seitenabschnitt hindurchführbar ist. Die Montageöffnung hat somit ausreichende Dimensionen, dass ein Hindurchführen des Handgriffs möglich ist. Dies erlaubt ein einfaches und sicheres Anbringen der Saugvorrichtung am Intubationsgerät, auch in Notfall- und Stresssituationen.

Gemäß einer vorteilhaften Ausgestaltung der Erfindung ist vorgesehen, dass der Hauptkörper eine Öffnung am patientennahen Ende im Bereich der wenigstens einen Ansaugöffnung hat. Dementsprechend kann die Absaugung von Flüssigkeiten durch die wenigstens eine Ansaugöffnung am patientennahen Ende durch den Hauptkörper hindurch erfolgen.

Gemäß einer vorteilhaften Ausgestaltung der Erfindung ist vorgesehen, dass der Hauptkörper überwiegend oder vollständig aus farblosem, transparentem Material besteht. Dies hat den Vorteil, dass durch den Anwender jederzeit durch visuelle Kontrolle geprüft werden kann, ob der gewünschte Absaugvorgang mit der Saugvorrichtung auch wie gewünscht abläuft, da die abgesaugten Flüssigkeiten dann visuell in dem Hauptkörper bzw. in darin verlegten Saugkanälen sichtbar sind. Hierdurch kann z.B. geprüft werden, ob der Unterdruck für die Absaugung ausreichend ist oder ob die wenigstens eine Ansaugöffnung verstopft ist.

Gemäß einer vorteilhaften Ausgestaltung der Erfindung ist vorgesehen, dass die Saugvorrichtung wenigstens einen sich von der wenigstens einen Ansaugöffnung bis zum patientenfernen Ende der Saugvorrichtung erstreckenden Saugkanal hat, der mit einem Anschluss zum Anschließen eines Unterdruckschlauchs verbunden ist. Dies hat den Vorteil, dass der Unterdruckschlauch eines medizinischen Vakuumsystems an einer Stelle angeschlossen werden kann, die während einer Intubation außerhalb des Patienten liegt. Vorteilhafter Weise kann ein solcher Saugkanal damit in den Hauptkörper integriert werden, sodass hierfür kein zusätzlicher Platzbedarf vorhanden ist und der Saugkanal auch nicht als Einzelteil störend im Wege ist. Ein solcher Saugkanal kann z.B. einstückig in wenigstens einem Seitenabschnitt und/oder in den Übergang zum Bodenabschnitt in dem Hauptkörper eingeformt werden. Es ist auch möglich, in dem Hauptkörper zur Realisierung des Saugkanals entlang wenigstens eines Seitenabschnitts und/oder des Bodenabschnitts einen Schlauch oder ein Rohr zu verlegen.

Gemäß einer vorteilhaften Ausgestaltung der Erfindung ist vorgesehen, dass die Saugvorrichtung wenigstens zwei nebeneinander angeordnete, voneinander unabhängige Saugkanäle hat, die jeweils eine Ansaugöffnung am patientennahen Ende der Saugvorrichtung haben und getrennt voneinander sich bis zum patientenfernen Ende der Saugvorrichtung erstrecken. Hierdurch kann ohne besonderen zusätzlichen Platzbedarf die Saugleistung der Saugvorrichtung deutlich erhöht werden. Zudem wird eine Redundanz bei der Absaugung geschaffen, die die Sicherheit der Saugvorrichtung erhöht. Beispielsweise ist weiterhin eine Absaugung von Flüssigkeiten möglich, selbst wenn einer der Saugkanäle verstopft werden sollte.

Gemäß einer vorteilhaften Ausgestaltung der Erfindung ist vorgesehen, dass die Saugvorrichtung eine Druckanzeigevorrichtung oder eine Befestigungsaufnahme für eine Druckanzeigevorrichtung hat, wobei durch die Druckanzeigevorrichtung der in dem wenigstens einen Saugkanal vorhandene Druck anzeigbar ist. Dies hat den Vorteil, dass direkt an der Saugvorrichtung der im wenigstens einen Saugkanal vorliegende Druck unmittelbar visuell dargestellt werden kann und dementsprechend vom Anwender auf einfache Weise überprüft werden kann, insbesondere ohne den Blick vom Patienten abzuwenden. Hierdurch erhält der Anwender jederzeit eine Information über den Druck im Saugkanal. Zusammen mit weiteren Indikatoren, wie z.B. dem Sauggeräusch und/oder der visuellen Überprüfung der transparenten Saugkanäle, erhält der Anwender auf einfache und intuitive Weise eine Information darüber, ob die Absaugung durch die Saugvorrichtung in Betrieb ist und ausreichende Saugwirkung hat.

Gemäß einer vorteilhaften Ausgestaltung der Erfindung ist vorgesehen, dass die Saugvorrichtung eine automatische Fehlererkennung hat, mit der Fehler in der Absaugfunktion der Saugvorrichtung anzeigbar sind. Dies hat den Vorteil, dass der Anwender durch eine automatische Funktion zusätzlich unterstützt wird und die Überprüfung der korrekten Absaugfunktion der Absaugvorrichtung nicht nur selbst z.B. anhand der zuvor erläuterten Indikatoren prüfen muss. Die automatische Fehlererkennung kann durch wenigstens eine mechanische Komponente an der Saugvorrichtung, wenigstens eine elektronische Komponente an der Saugvorrichtung und/oder eine Softwarefunktion realisiert werden, z.B. eine Softwarefunktion in einem Anzeigesystem des Intubationsgeräts.

Die der zuvor erläuterten Ausgestaltungen des Hauptkörpers der Saugvorrichtung sind auch als jeweilige unabhängige Erfindung anzusehen, insbesondere unabhängig von der Platzierung der wenigstens einen Ansaugöffnung.

Die eingangs genannte Aufgabe wird außerdem gelöst durch ein Intubationsgerät zum Intubieren eines Patienten mit einem Endotrachealtubus, mit folgenden Merkmalen:
a) einem Handgriff zum Halten des Intubationsgeräts,
b) eine mit dem Handgriff verbundene Führungsschiene, die einen in Längsrichtung von einem patientenfernen Ende bis zu einem patientennahen Ende der Führungsschiene verlaufenden Führungskanal zum Führen des Endotrachealtubus und eine beim Intubationsvorgang der Zunge des Patienten zugewandte Oberseite hat,
c) eine Saugvorrichtung, die zum Absaugen von Flüssigkeiten im Bereich des patientennahen Endes des Intubationsgeräts eingerichtet ist,
d) wobei die Saugvorrichtung wenigstens eine Ansaugöffnung zum Ansaugen der Flüssigkeiten hat, die relativ zu einer beim Intubationsvorgang der Zunge des Patienten zugewandten Oberseite der Führungsschiene an einer Position unterhalb der wenigstens einen optischen Erfassungsvorrichtung oder unterhalb der Führungsschiene angeordnet ist, wenn Saugvorrichtung am Intubationsgerät befestigt ist.

Auch hierdurch können die zuvor erläuterten Vorteile realisiert werden. Die Saugvorrichtung kann baulich in das Intubationsgerät integriert sein, z.B. wenigstens zum Teil in die Führungsschiene integriert sein. Beispielsweise kann der wenigstens eine Saugkanal an der Führungsschiene angebracht sein oder innen in die Führungsschiene integriert sein.

Gemäß einer vorteilhaften Ausgestaltung der Erfindung ist vorgesehen, dass die Führungsschiene zumindest in einem patientennahen Bereich einen Biegeabschnitt aufweist, in dem die Führungsschiene von der Seite aus betrachtet, an der der Handgriff angeordnet ist, konvex gebogen ist. Der Biegeabschnitt kann z.B. einen 90 Grad-Bogen umfassen, oder einen Bogen von etwas mehr oder weniger als 90 Grad, z.B. etwa 80 Grad. Vorteilhaft ist z.B. ein Biegeabschnitt, der sich über einen Bogen von 70 bis 90 Grad erstreckt. Dies ist förderlich für eine zielgerichtete, zügige Einführung der Führungsschiene in die Atemwege eines Patienten, so dass der Patient bestmöglich geschont wird.

Gemäß einer vorteilhaften Ausgestaltung der Erfindung ist vorgesehen, dass die Führungsschiene am patientenfernen Ende des Biegeabschnitts in einen linear verlaufenden oder im Vergleich zum Biegeabschnitt weniger stark gekrümmten Abschnitt übergeht. Dies ermöglicht insbesondere eine gleitende Linearführung oder quasi-lineare Führung des Epiglottishebers auf der Führungsschiene. Dabei kann die Führungsschiene im weniger stark gekrümmten Abschnitt einen wesentlich größeren Biegeradius haben als im Biegeabschnitt.

Gemäß einer vorteilhaften Ausgestaltung der Erfindung ist vorgesehen, dass das Intubationsgerät einem Epiglottisheber zum Anheben der Epiglottis des Patienten hat, wobei der Epiglottisheber über wenigstens ein Lagerungselement an einem Bauteil des Intubationsgeräts beweglich gelagert ist. Hiermit wird ein Intubationsgerät geschaffen, das ein zügiges, sicheres und patientenschonendes Intubieren auch unter Narkose erlaubt.

Gemäß einer vorteilhaften Ausgestaltung der Erfindung ist vorgesehen, dass der Führungskanal auf der Innenseite des Biegeabschnitts überwiegend oder vollständig offen ist. Auch dies ist förderlich für eine flachbauende Konstruktion des Intubationsgerätes. Der Führungskanal kann auf der Oberseite auch außerhalb des Biegeabschnitts überwiegend oder vollständig offen sein, auch über die gesamte Länge. Insbesondere kann die Führungsschiene zumindest im Biegeabschnitt oder insgesamt ein U-förmiges Profil haben.

Gemäß einer vorteilhaften Ausgestaltung der Erfindung ist vorgesehen, dass der Epiglottisheber zumindest im patientennahen Bereich, insbesondere in einem Biegeabschnitt der Führungsschiene, die Führungsschiene oder zumindest den Führungskanal auf der Oberseite der Führungsschiene überwiegend oder vollständig geschlossen überdeckt. Der Epiglottisheber ist somit in dem Bereich, in dem er die Führungsschiene oder zumindest den Führungskanal auf der Oberseite der Führungsschiene überdeckt, überwiegend oder vollständig geschlossen ausgebildet und bildet hierdurch ein Dach über der wenigstens einen optischen Erfassungseinrichtung im patientennahen Bereich an der Führungsschiene und/oder der wenigstens einen optischen Erfassungseinrichtung im patientennahen Bereich an dem Epiglottisheber. Auf diese Weise ist zumindest die Erfassungsseite der optischen Erfassungseinrichtung, z.B. eine Linse, ein Objektiv oder eine Kamera, unterhalb des überwiegend oder vollständig geschlossenen Bereichs des Epiglottishebers angeordnet (sozusagen unter dem Dach) und dadurch vor einer Sichtblockade durch Zungengewebe oder durch Verschmutzung geschützt.

### Lösungen für die oben erläuterten neun Problembereiche

Die hier beschriebene Saugvorrichtung ermöglicht vor und während der videolaryngoskopischen Intubation eine zielgerichtete und sichere Absaugung. Die Saugvorrichtung kann sowohl intuitiv am Intubationsgerät angebracht als auch intuitiv bedient (s.u.) werden und ist somit auch für Notfall- und Stresssituationen geeignet:

### Vorteile der Saugvorrichtung im Vergleich zu an Spateln befestigten Absaugschläuchen

### 1. Keine Unterbrechung des Sogs durch Abknicken eines flexiblen Absaugschlauches

Die Saugvorrichtung weist rigide Saugkanäle auf: Entweder wird hierüber abgesaugt oder es werden flexible Schläuche in diesen Kanälen eingelegt. Ein Abknicken ist ausgeschlossen.

### 2. Vermeidung einer unsicheren Führung durch freie Beweglichkeit eines flexiblen Absaugschlauches

Wie oben unter 1. beschrieben sind die Saugkanäle bzw. Absaugschläuche innerhalb der Saugvorrichtung fixiert. Eine freie Beweglichkeit und damit unsichere Führung ist dadurch ausgeschlossen.

### 3. Automatische Führung in die optimale Saugerposition und Halten dieser Position durch unabhängiges Öffnen der Atemwege

### 3.1. Automatische Führung in die optimalen Saugerposition

Die für eine optimale Sicht auf den Kehlkopfeingang und somit für die Intubation erforderliche Intubationsgerät-Position stellt gleichzeitig auch die optimale Position für die Ansaugöffnungen der Saugvorrichtung dar: Diese Ansaugöffnungen liegen dann - ohne darüber nachzudenken - ganz automatisch gezielt und verlässlich am tiefsten Punkt unter dem Kehlkopf direkt vor der Speiseröhre, wo sich Flüssigkeit in den Atemwegen ansammelt. So kann die dort sich ansammelnde und dann schließlich aufsteigende Flüssigkeit abgesaugt werden, bevor sie Kehlkopfeingang und Optiken erreichen kann.

### 3.2. Automatisches Halten der optimalen Saugerposition durch unabhängiges Öffnen der Atemwege

Die Kontinuität dieser optimalen Absaugposition wird automatisch durch die unabhängige Öffnung der Atemwege gewährleistet:
Im Gegensatz zu allen anderen Videolaryngoskopiesystemen verbleiben die optische Erfassungseinrichtung und die Führungsschiene des Intubationsgeräts vor und während der Intubation in der für die Sicht und die Tubusführung optimalen Position unterhalb des Kehlkopfdeckels (Epiglottis). Das wird durch die unabhängige Öffnung der Atemwege mit dem Epiglottislifter am Intubationsgerät erreicht: Wenn der Epiglottislifter angehoben wird, um den Kehlkopfbereich zu eröffnen und um so eine optimale Sicht für die Intubation zu erreichen, wird die unter dem Intubationsgerät befestigte Saugvorrichtung ebenfalls nicht bewegt und verbleibt somit in der optimalen Position unter dem Kehlkopf direkt vor der Speiseröhre.

### 4. Von der Tubusposition unabhängige Führung der Absaugung

Da kein Absaugschlauch durch den Tubus geführt werden muss, ist die Absaugung unabhängig von der Position des Tubus. Somit kann während der gesamten Intubation unter optimaler Sicht unterhalb des Kehlkopfes abgesaugt werden.

### 5. Vereinfachte Absaugaktivierung durch einhändige Steuerung von Laryngoskop und Saugwirkung (Sog)

Die Aktivierung des Sogs ist einfach und intuitiv, so dass keine Hilfe durch eine zweite Person erforderlich ist.

Die zu verschließende Öffnung zur Aktivierung der Saugwirkung wird durch die Saugvorrichtung fest und sicher am vorderen Teil des Intubationsgerät Griffes positioniert. So kann dann mit dem Daumen der gerätesteuernden Hand standardmäßig die Absaugung einfach und intuitiv aktiviert werden, so dass die zweite Hand für eine Intubation oder eine andere Intervention zur Verfügung steht.

Mit einer Hand kann also das Gerät gesteuert und gleichzeitig die Absaugung aktiviert werden. Hierdurch wird die bedarfsgerechte und gezielte, videolaryngoskopische Absaugung unter Sicht sehr vereinfacht und dadurch sicherer:
Ein Ansaugen der Schleimhäute mit entsprechendem Saugverlust bei der sonst meist üblichen kontinuierlichen Aktivierung der Saugung ist somit besser zu vermeiden.

### 6. Kein Sogverlust durch mangelnde Führung eines flexiblen Absaugschlauches

Wie oben bereits erläutert, wird kein flexibler Absaugschlauch verwendet, sondern vielmehr stabile Saugkanäle innerhalb bzw. an der Saugvorrichtung. Dadurch wird wie oben unter 1. und 2. beschrieben sowohl eine Vermeidung von Sogverlust durch Abknicken als auch die sichere Führung zum tiefsten Punkt unterhalb des Kehlkopfes durch nur eine Person mit der geräteführenden Hand gewährleistet.

Darüber hinaus kann durch die einfache und sichere Führung der Saugvorrichtung unter optischer Kontrolle des Intubationsgeräts besser vermieden werden, dass Ausbuchtungen und Taschen im Bereich der oberen Atemwege sich um die Ansaugöffnungen legen. Das würde zu einem Verlust des Soges führen, so dass Flüssigkeiten nicht mehr abgesaugt werden können.

Auch bei einem rigiden Sauger kann so ein Saugverlust nach Ansaugen bei mangelnder Kontrolle der Führung auftreten.

### Vorteile der Saugvorrichtung im Vergleich zu einem zusätzlich eingeführten Sauger

### 7. Keine Behinderung der Intubation durch einen rechts eingeführten, rigiden Sauger, leichtes Halten der optimalen Saugerposition und leichtes Verhindern von Ansaugen

### 7.1. Keine Behinderung der Intubation wie bei einen rechts zusätzlich eingeführten, rigiden Sauger

Durch das Intubationsgerät und die daran befestigte Saugvorrichtung entsteht keine räumliche Enge oder direkte Behinderung der Tubusführung, die die Intubation erschweren könnte. Die Hilfe einer zweiten Person, die den rigiden Sauger rechts außen halten muss, ist nicht erforderlich.

Dies wird dadurch erreicht, dass die Saugvorrichtung z.B. mit zwei Absaugschläuchen mit einem Außendurchmesser von jeweils 4,7 mm ausgebildet sein kann und direkt unter der Führungsschiene des Intubationsgeräts befestigt und dann mit dem Intubationsgerät fest verbunden werden kann. Dadurch bleibt der Raum oberhalb der Führungsschiene des Intubationsgeräts frei. So werden das gezielte Vorschieben des Tubus und die Intubation nicht durch die Saugvorrichtung negativ beeinflusst.

Ein kompliziertes, synchronisiertes Vorgehen zwischen zwei Personen ist nicht erforderlich und muss dementsprechend auch nicht geübt werden.

### 7.2. Leichtes und automatisches Halten der optimalen Saugerposition

Im Gegensatz zu einem zusätzlich eingeführten rigiden Sauger gibt es bei der Saugvorrichtung weder Probleme bei der korrekten Positionierung der Ansaugöffnung noch beim Halten dieser optimalen Position am tiefsten Punkt. Wie oben unter 3. bereits ausführlich erläutert, wird die optimale Saugerposition automatisch mit der geräteführenden Hand erreicht und dann auch so gehalten, sobald auch die Sicht auf den Kehlkopfeingang für die Intubation eingestellt und aufrechterhalten wird.

Hierbei besteht also im Gegensatz zu einem zusätzlich eingeführten rigiden Sauger eine gute Kontrolle über die Position der beiden Absaugöffnungen, die sich direkt unterhalb der unteren Bildkante - also unterhalb der Aryknorpel - befinden.

### 7.3. Leichteres Verhindern von Festsaugen an der Schleimhaut

Im Gegensatz zu einem zusätzlich rechts neben dem Videolaryngoskop eingeführten rigiden Sauger durch die laryngoskopierende oder eine weitere Person wird bei der Saugvorrichtung nicht vorsorglich ein kontinuierlicher Sog aktiviert. Das verhindert sicher das Festsaugen an der Schleimhaut, da der Sog an den Saugeröffnungen ohne Verschluss der Aktivierungsöffnung auf dem Intubationsgerät-Handgriff sehr stark reduziert ist. Erst wenn Flüssigkeit vorhanden ist und sich vor den Saugeröffnungen befindet, wird der Sog aktiviert und die Flüssigkeit effektiv entfernt.

Darüber hinaus wird die Positionierung der Ansaugöffnungen wie oben bereits mehrfach beschrieben genau durch die geräteführende Hand kontrolliert: Die Ansaugöffnungen befinden sich automatisch zwar vor dem Kehlkopf direkt unterhalb der Aryknorpel. Allerdings muss zur Erlangung einer optimalen Sicht und Tubusführung das Intubationsgerät mit der darunter befestigten Saugvorrichtung leicht von der hinteren Rachenschleimhaut angehoben werden. Daher besteht in der Regel kein direkter Kontakt der nach unten und vorn gerichteten Ansaugöffnungen zu der hinteren Rachenschleimhaut. Hierdurch kann ein Ansaugen im Vergleich zu der schwer zu kontrollierenden Saugeröffnung eines zusätzlich und unabhängig vom Videolaryngoskops geführten, rigiden Saugers besser verhindert werden.

Darüber hinaus können z.B. zwei im Durchmesser 4,7 mm große Ansaugöffnungen mit der Saugvorrichtung direkt an den optimalen Punkt geführt werden. Das erhöht die Wahrscheinlichkeit, dass zumindest eine Ansaugöffnung offen bleibt. Falls doch der sehr unwahrscheinliche Fall eintreten sollte, dass sogar beide Ansaugöffnungen durch Ansaugen von Schleimhaut, zähem Sekret, Blutkoageln, Nahrungs- oder Gewebestücke verstopft werden, würde das eine in die Saugvorrichtung integrierte, dann ausschlagende Druckanzeige in Zusammenhang mit fehlendem Aspirat direkt anzeigen. Dann kann die Position der Saugvorrichtung mit dem Intubationsgerät korrigiert oder das Gerät ganz entfernt und die angesaugten Stücke entfernt werden.

### 8. Kein blindes Einführen wie bei einem links des Laryngoskopes zusätzlich eingeführten, rigiden Sauger

Im Gegensatz zu einem links des Videolaryngoskopes ohne Sicht eingeführten rigiden Sauger wird die Saugvorrichtung wie oben bereits ausführlich erläutert immer automatisch gezielt und unter optischer Kontrolle an den optimalen Punkt geführt. Hierdurch wird das Risiko des Ansaugens mit einer nachfolgenden Reduktion oder einem Verlust des Sogs minimiert. Ein umständliches Umfassen des links eingeführten Saugers und des Videolaryngoskopes mit der linken Hand entfällt.

### 9. Keine mangelnde oder fehlende Kontrolle über die Saugeffizienz

Im Gegensatz zu dem bisherigen Stand der Technik kann über eine Druckanzeige an der Saugvorrichtung direkt und sicher die Saugereffizienz während der Laryngoskopie kontrolliert werden.

### 5. Eigenschaften der Saugvorrichtung

Die Saugvorrichtung muss in verschiedenen Phasen des Gebrauchs unterschiedliche Funktionen gewährleisten: Diese fünf Phasen des Gebrauchs sind: 1. Bereitstellung, 2. Einführen, 3. Laryngoskopie, 4. Intubation und 5. Entfernen aus dem Mund. Um hierbei die Lösungen der Problembereiche gewährleisten zu können, kann die für das Intubationsgerät konzipierte Saugvorrichtung in diesen verschiedenen Phasen des Gebrauchs folgende Eigenschaften aufweisen:

### 5.1.-5.5. Befestigung, Verbindung und Ausrichtung der Saugvorrichtung mit bzw. an dem Intubationsgerät

5.1. Die Saugvorrichtung kann leicht, rasch und intuitiv am Intubationsgerät sowohl anzubringen als auch zu entfernen sein.

5.2. Die Saugvorrichtung kann dann fest und sicher am Intubationsgerät befestigt sein: Eine versehentliche Loslösung von dem Intubationsgerät kann in allen Phasen des Gebrauchs sicher ausgeschlossen sein.

5.3. Nach der Befestigung können sowohl die Ansaugöffnungen als auch die Öffnung zur Aktivierung des Sogs automatisch optimal bei Gebrauch des Intubationsgeräts positioniert und ausgerichtet sein.

5.4. Diese optimale Ausrichtung kann in allen Phasen des Gebrauchs unbeeinträchtigt und konstant sein.

5.5. Die bedarfsgerechte Aktivierung des Sogs kann leicht, intuitiv und rasch mit der geräteführenden Hand erfolgen können - ohne die Hilfe einer zweiten Person. Dadurch wird die Aktivierung der Saugung automatisch mit der Geräteführung unter Sicht synchronisiert.

### 5.6.-5.8. Minimierung oder Vermeidung des Risikos einer eingeschränkten Funktionsfähigkeit durch den Gebrauch

5.6. Das Risiko einer eingeschränkten Funktionsfähigkeit durch Abknicken des Sauglumens bei Einführen, Laryngoskopie und Intubation und ein hierdurch verursachter Verlust der Saugleistung ist minimiert.

5.7. Das Risiko einer eingeschränkten Funktionsfähigkeit durch Ansaugen an die Schleimhaut und ein hierdurch verursachter Verlust der Saugleistung ist minimiert.

5.8. Das Risiko einer eingeschränkten Funktionsfähigkeit durch Ansaugen von größeren festen Bestandteilen, die nicht durch das Sauglumen abtransportiert werden können - wie wie z.B. Nahrungsreste, zäher Schleim, Gewebestücke eines zerfallenen Tumors oder Blutkoagel und ein hierdurch verursachter Verlust der Saugleistung ist minimiert.

### 5.9.-5.11. Minimierung oder Vermeidung des Risikos einer eingeschränkten Funktionsfähigkeit des Intubationsgeräts durch die Saugvorrichtung

5.9. Die Saugvorrichtung darf den Gesamtdurchmesser des Intubationsgeräts nur so wenig wie möglich erhöhen, um vor allem das Einführen in den Patienten so wenig wie möglich zu behindern.

5.10. Die Saugvorrichtung darf das Vorschieben des Tubus von der Intubationsgerät Schiene in den Kehlkopfeingang nicht behindern.

5.11. Die Saugvorrichtung darf die Positionierung des Epiglottislifters auf dem Intubationsgerät und seine Betätigung nicht behindern.

5.12. Die Saugvorrichtung kann eine optische und/oder akustische Anzeige aufweisen, mit der eine sichere Kontrolle über die erzielte Saugleistung gewährleistet wird.

Die Erfindung wird nachfolgend anhand von Ausführungsbeispielen unter Verwendung von Zeichnungen näher erläutert.

Es zeigen
- Figur 1: eine Saugvorrichtung in perspektivischer Ansicht,
- Figur 2: die Saugvorrichtung in Seitenansicht,
- Figur 3: die Saugvorrichtung ein einer Ansicht von oben,
- Figur 4: die Saugvorrichtung in einer Ansicht von vorn,
- Figur 5: ein Intubationsgerät in perspektivischer Ansicht,
- Figur 6: das Intubationsgerät in Seitenansicht mit einem Epiglottisheber,
- Figur 7: das Intubationsgerät mit daran angebrachter Saugvorrichtung in perspektivischer Ansicht,
- Figur 8: der patientennahe Bereich der Anordnung gemäß Figur 7 in einer Ansicht von vorn,
- Figur 9: die Anordnung gemäß Figur 7 in einer Ansicht von oben,
- Fig. 10-13: Schritten der Anbringung der Saugvorrichtung am Intubationsgerät in Seitenansicht.

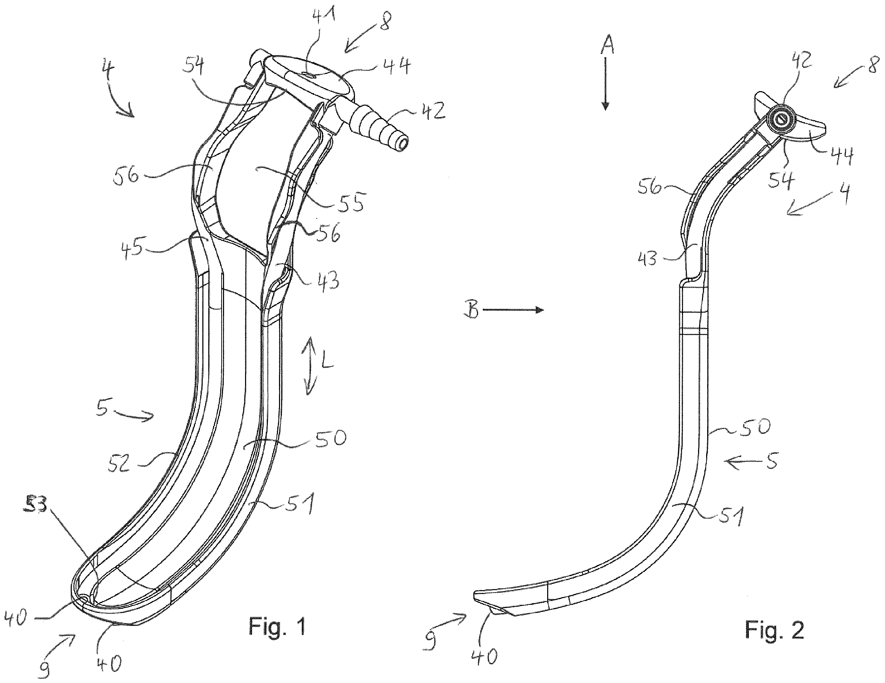

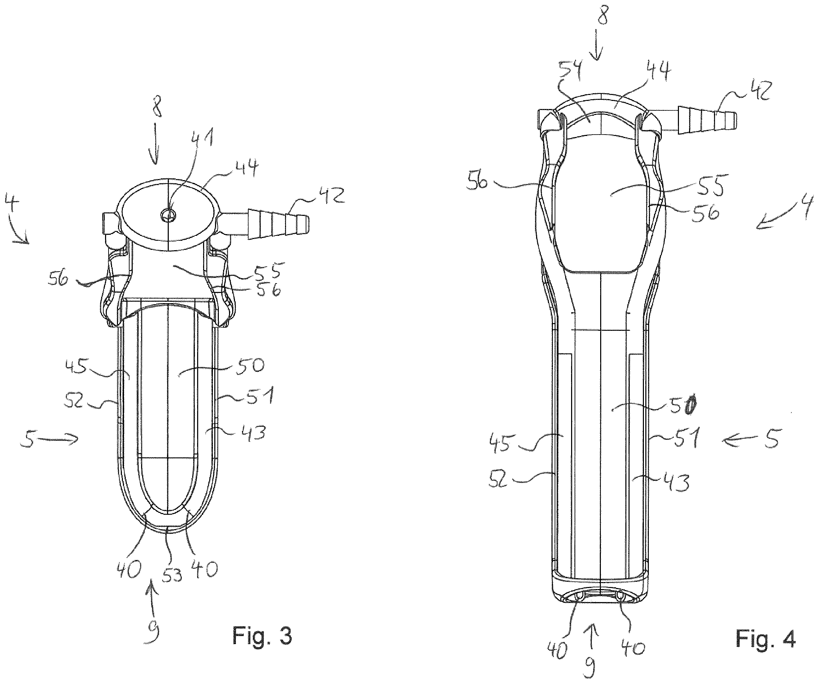

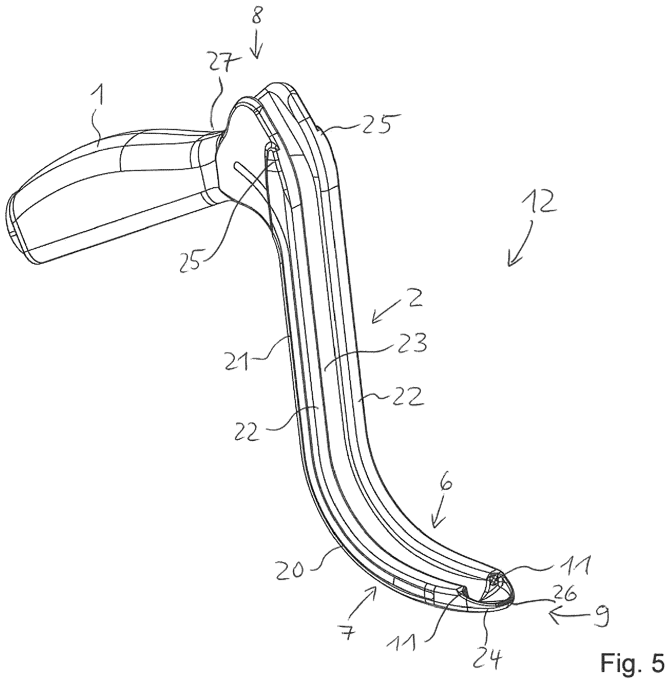

Die Figuren 1 bis 4 zeigen eine Saugvorrichtung 4, die als separates Bauteil von einem in Figur 5 dargestellten Intubationsgerät 12 ausgebildet ist, in verschiedenen Ansichten. Die Figur 3 zeigt die Saugvorrichtung 4 in der in Figur 2 markierten Betrachtungsrichtung A, die Figur 4 zeigt die Saugvorrichtung 4 in der in Figur 2 markierten Betrachtungsrichtung B. Die Saugvorrichtung 4 hat einen Hauptkörper 5, der in seiner Längserstreckungsrichtung L gekrümmt ausgebildet ist und insbesondere in diesem gekrümmten Bereich einen gekrümmten Verlauf der Führungsschiene 2 des Intubationsgeräts 12 folgt, wie nachfolgend noch dargestellt wird.

Der Hauptkörper 5 hat am patientennahen Ende 9 ein patientennahes Befestigungselement 53, mit dem der Hauptkörper 5 an einer ersten Befestigungsstelle 26 am patientennahen Ende 9 der Führungsschiene 2 anrastbar ist. Der Hauptkörper 5 hat außerdem ein patientenfernes Befestigungselement 54, mit dem der Hauptkörper 5 an einer zweiten Befestigungsstelle 27 am patientenfernen Ende 8 des Intubationsgeräts 12, insbesondere an der Führungsschiene 2 und/oder dem Handgriff 1, befestigbar ist.

Der Hauptkörper 5 hat einen ersten Seitenabschnitt 51 und einen davon beabstandeten zweiten Seitenabschnitt 52, wobei diese Seitenabschnitte 51, 52 wie seitliche Stege in Längsrichtung L verlaufen und dem gekrümmten Verlauf des Hauptkörpers 5 folgen. Die Seitenabschnitte 51, 52 sind im patientennahen Bereich 9 miteinander zusammengeführt oder über einen Quersteg miteinander verbunden. Die Seitenabschnitte 51, 52 sind im mittleren Bereich über einen Bodenabschnitt 50 miteinander verbunden. Der Hauptkörper 5 hat in diesem Bereich eine durch die Seitenabschnitte 51, 52 und den Bodenabschnitt 50 gebildete U-Form, in die zumindest ein Teil der Führungsschiene 2 des Intubationsgeräts 12 eingesetzt werden kann. Der Bodenabschnitt 50 endet noch vor dem patientenfernen Ende 8 des Hauptkörpers 5, wobei in diesem Bereich die Seitenstege 51, 52 in jeweils einen Abschnitt 56 übergehen. Die Abschnitte 56 umranden eine Montageöffnung 55 seitlich. Bei der Anbringung der Saugvorrichtung 4 am Intubationsgerät 12 kann der Handgriff 1 und/oder die Führungsschiene 2 des Intubationsgeräts 12 durch die Montageöffnung 55 zwischen dem ersten und dem zweiten Seitenabschnitt 51, 52 hindurch geführt werden.

Am patientenfernen Ende 8 ist am Hauptkörper 5 eine Baugruppe 44 angeordnet, die mehrere Funktionen hat. So ist an der Baugruppe 44 an der zum patientennahen Ende 9 gewandten Seite das patientenferne Befestigungselement 54 ausgebildet. Die Baugruppe 44 sorgt zudem für eine zusätzliche mechanische Stabilität des Hauptkörpers 5 im patientenfernen Bereich 8, indem durch die Baugruppe 44 die Abschnitte 56 miteinander verbunden sind. Die Montageöffnung 55 ist somit von der Baugruppe 44, den Abschnitten 56 und dem Rand des Bodenabschnitts 50 umrandet.

Zusätzlich ist an der Baugruppe 44 ein Anschluss 42 zum Anschluss eines Unterdruckschlauchs angeordnet. Durch den Unterdruckschlauch kann ein Unterdruck in das Innere der Baugruppe 44, die einen Hohlraum aufweist, eingeleitet werden. Die Baugruppe 44 ist über einen ersten Saugkanal 43 und zusätzlich durch einen getrennt vom ersten Saugkanal 43 ausgebildeten zweiten Saugkanal 45 mit dem patientennahen Ende 9 verbunden. Die Saugkanäle 43, 45 enden mit einer jeweiligen Ansaugöffnung 40 in diesem patientennahen Bereich 9. Durch die Baugruppe 44 kann der über den Anschluss 42 eingeleitete Unterdruck beiden Saugkanälen 43, 45 zugeführt werden. Angesaugte Flüssigkeiten werden dann an den Ansaugöffnungen 40 angesaugt, durch die Saugkanäle 43, 45 zur Baugruppe 44 geführt und über den Anschluss 42 abgeführt.

An der Baugruppe 44 ist zudem ein Betätigungselement 41 vorhanden, mit dem der Anwender mit einem Finger die Stärke der Absaugwirkung an den Ansaugöffnungen 40 beeinflussen kann. Das Bedienelement 41 kann beispielsweise als eine Öffnung mit definiertem relativ kleinem Querschnitt ausgebildet sein, die einen Zugang zum inneren Hohlraum in der Baugruppe 44 bereitstellt. Solange diese Öffnung 41 mit dem Finger nicht abgedeckt ist, ist die Saugwirkung an den Ansaugöffnungen 40 relativ gering, da stattdessen Luft aus der Umgebung durch die Öffnung 41 angesaugt wird. Wird die Öffnung 41 mit dem Finger vollständig oder teilweise verschlossen, kann durch den Anwender auf diese Weise dosiert die Ansaugwirkung an den Ansaugöffnungen 40 erhöht und nach Wunsch variiert werden.

Vorteilhafter Weise sind die Saugkanäle 43, 45 baulich so in den Hauptkörper 5 integriert, dass sie nicht ungewollt komprimiert und dadurch verschlossen werden können. Dennoch verbleibt ausreichend Bauraum, um die Saugvorrichtung bzw. den Hauptkörper 5 an der Unterseite 7 der Führungsschiene 2 des Intubationsgeräts 12 anzurasten und dabei zumindest den unteren Teil der Führungsschiene 2 in dem U-förmigen Bereich des Hauptkörpers 5 aufzunehmen.

Die Figur 5 zeigt ein Intubationsgerät 12, das als Videolaryngoskop ausgebildet ist. Das Intubationsgerät 12 hat einen Handgriff 1 zum Halten des Intubationsgeräts 12. Der Handgriff 1 kann besonders ergonomisch ausgeformt sein, z.B. mit einem entsprechenden Profil, das gut gegriffen werden kann und nicht so leicht verrutscht. Mit dem Handgriff 1 kann das Intubationsgerät 12 insbesondere während der Intubation geführt werden. Der Handgriff bildet eine definierte Griffstelle zum Greifen und Halten des Intubationsgeräts 12. Der Handgriff 1 ist dazu eingerichtet, dass er vom Anwender mit der ganzen Hand gegriffen werden kann.

Das Intubationsgerät 12 hat außerdem eine starre Führungsschiene 2, die ebenfalls starr mit dem Handgriff 1 verbunden ist. Der Handgriff 1 ragt von einer Unterseite 7 der Führungsschiene 2 ab und bildet zu der Unterseite 7 der Führungsschiene 2 einen Winkel von weniger als 90°, z.B. im Bereich von 70°. Der Handgriff 1 ist auf diese Weise zum patientennahen Bereich 8 der Führungsschiene 2 hin etwas schräggestellt. Die Führungsschiene 2 hat einen in Längsrichtung von dem patientenfernen Ende 8 bis zu dem patientennahen Ende 9 der Führungsschiene 2 verlaufenden Führungskanal 23 zum Führen des Endotrachealtubus.

Im patientennahen Bereich 9 endet die Führungsschiene 2 mit einem Biegeabschnitt 20, in dem die Führungsschiene 2 in Seitenansicht mit einem gewissen Radius gekrümmt ausgebildet ist. Nahe des freien Endes 24 der Führungsschiene 2, d.h. an der Spitze der Führungsschiene 2, ist wenigstens eine optische Erfassungseinrichtung 11 in die Führungsschiene 2 integriert. Beispielsweise kann nebeneinander auf gegenüberliegenden Seiten des Führungskanals 23 jeweils eine optische Erfassungseinrichtung 11 in die Führungsschiene 2 integriert sein. Der Biegeabschnitt 20 kann sich über einen Winkelbereich von z.B. 70° bis 100° erstrecken, wobei ein Winkel von etwas unter 90° besonders vorteilhaft ist. Vom patientennahen Bereich 9 aus gesehen schließt sich an den Biegeabschnitt 20 in der Seitenansicht gerade verlaufender Bereich 21 der Führungsschiene an. Dieser gerade verlaufende Bereich 21 mündet in den Handgriff 1. Die Führungsschiene 2 ist im Querschnitt U-förmig ausgebildet und dementsprechend zur Oberseite 6 hin offen. Die Oberseite 6 der Führungsschiene 2 ist beim Intubationsvorgang der Zunge des Patienten zugewandt. Über die gesamte Längserstreckung der Führungsschiene 2 verläuft darin ein Führungskanal 23, in dem ein Endotrachealtubus entlang der Führungsschiene 2 geführt werden kann. Der Führungskanal 23 ist links und rechts von erhabenen Randseiten der Führungsschiene 2 begrenzt.

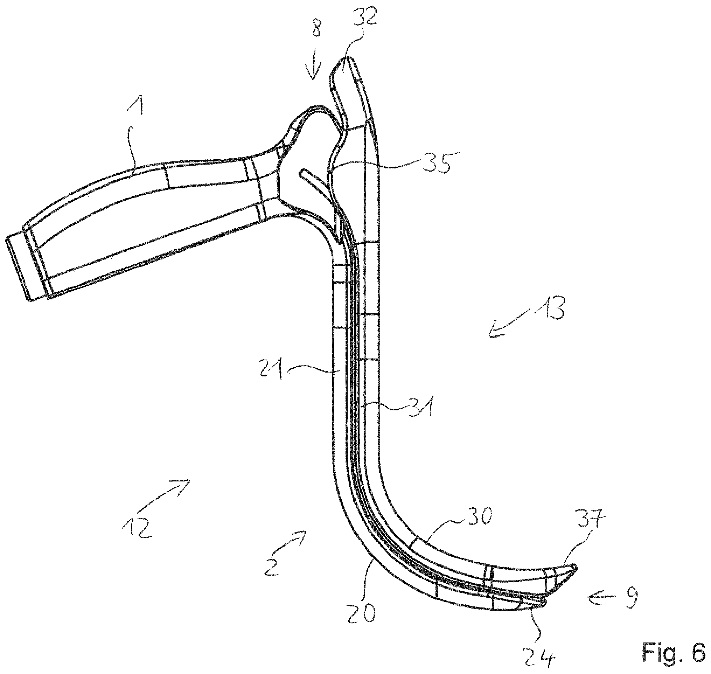

An der Oberseite 6 der Führungsschiene 2 kann ein Epiglottisheber 3 angeordnet werden, wie die Figur 6 zeigt. Die den Führungskanal 23 ist links und rechts begrenzenden erhabenen Randseiten der Führungsschiene 2 bilden mit Ihren zum Epiglottisheber 3 gewandten Oberflächen Auflagerungsflächen 22 zur Auflagerung des Epiglottishebers 3. Der Epiglottisheber 3 kann hinsichtlich seiner Formgebung an die Formgebung der Führungsschiene 2 angepasst sein, d.h. er folgt der Formgebung der Führungsschiene 2, sodass der Epiglottisheber 3 im gerade verlaufenden Bereich 21 der Führungsschiene 2 ebenfalls im Wesentlichen gerade verlaufend ausgebildet ist. Im Biegeabschnitt 20 der Führungsschiene 2 ist der Epiglottisheber 3 in analoger Weise gebogen ausgebildet. Erst am patientennahen freien Ende kann der Epiglottisheber 3 mit einer anderen Formgebung fortgeführt werden, insbesondere kann er über das freie Ende 24 der Führungsschiene 2 im patientennahen Bereich 9 etwas hinausstehen, z.B. indem dort eine Spatelspitze 37 ausgebildet ist. Am Intubationsgerät 12, insbesondere an der Führungsschiene 2, können formschlüssige Befestigungselemente 25 angeordnet sein, die mit formschlüssigen Befestigungselementen 35 des Epiglottishebers 3 zusammenwirken, um diesen an der Führungsschiene 2 zu halten.

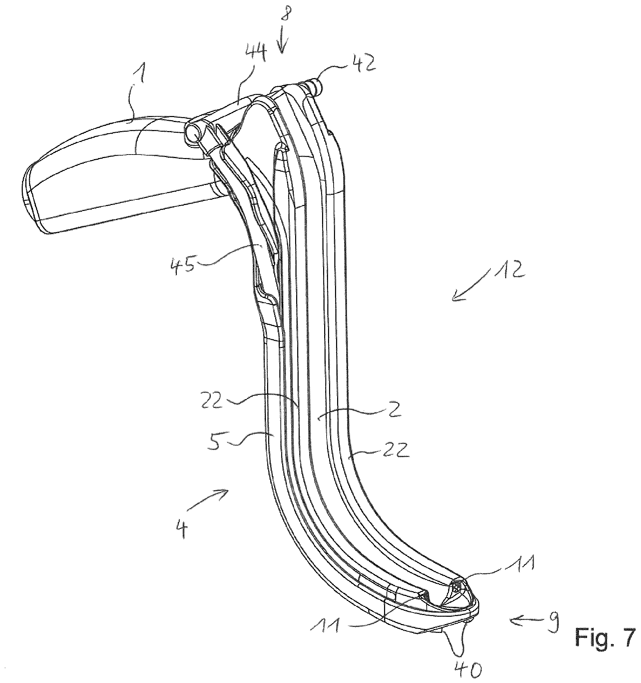

Die Figur 7 zeigt das Intubationsgerät 12 mit der daran befestigten Saugvorrichtung 4. In diesem Zustand liegt das patientennahe Befestigungselement 53 an der ersten Befestigungsstelle 26 an der Führungsschiene 2 an. Das patientenferne Befestigungselement 54 liegt an einer zweiten Befestigungsstelle 27 an der Rückseite der Führungsschiene 2 und/oder am Handgriff 1 an. Die U-förmige Führungsschiene 2 ist dann zumindest zum Teil in den U-förmigen Abschnitt der Saugvorrichtung 4 bzw. des Hauptkörpers 5 eingebettet, sodass die erhabenen Randseiten der Führungsschiene 2 an den Seitenabschnitten 51, 52 angeordnet sind und dort eingebettet sind. Man erkennt in der Figur 7 zudem gut die weit unten liegende Anordnung der Ansaugöffnungen 40, die unterhalb der optischen Erfassungseinrichtung 11 angeordnet sind.

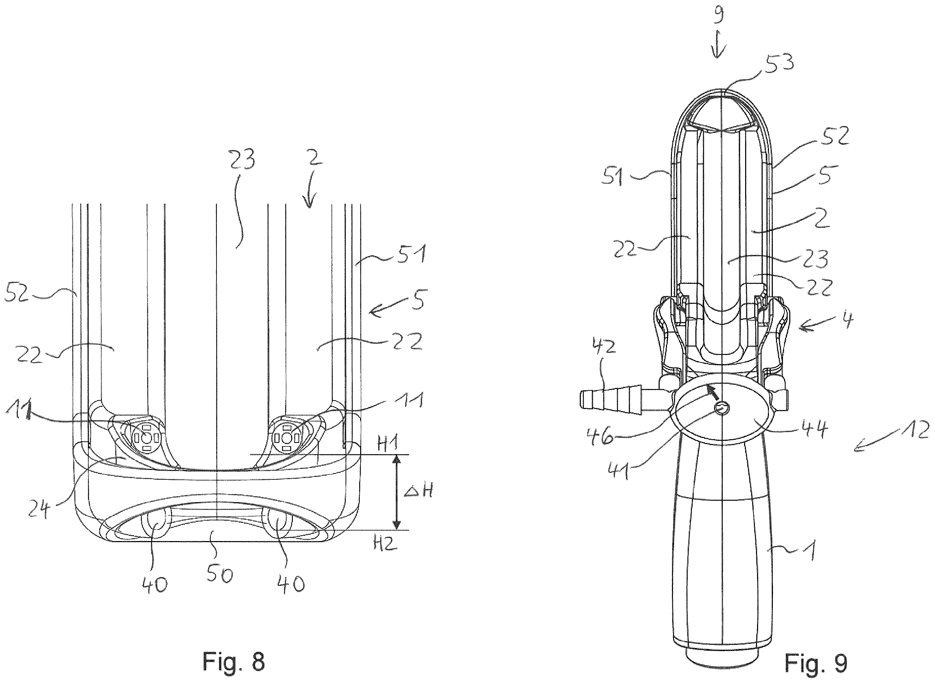

Die Figur 8 ist eine Draufsicht auf den patientennahen Bereich des Intubationsgeräts 12 in der Blickrichtung B, wobei wie in Figur 7 die Saugvorrichtung 4 am Intubationsgerät 12 befestigt ist. Durch die mit der Kennzeichnung H1 markierte Linie wird die Höhenlage der optischen Erfassungseinrichtung 11 markiert. Die Linie H2 markiert die Höhenlage der wenigstens einen Ansaugöffnung 40. Man erkennt, dass die Ansaugöffnung 40 an das Maß ΔH unterhalb der optischen Erfassungseinrichtung 11 angeordnet ist. Auf diese Weise wird eine relativ weit unten liegende Absaugposition realisiert, die unterhalb der optischen Erfassungseinrichtung 11 liegt, sodass eine Absaugung von Flüssigkeiten bereits durchgeführt werden kann, bevor diese Flüssigkeiten das Höhenniveau H1 erreichen. Auf diese Weise kann die optische Erfassungseinrichtung 11 auch ohne Spülsystem gut sauber gehalten werden.

Die Figur 9 zeigt das Intubationsgerät 12 mit der daran angebrachten Saugvorrichtung 4 in der Betrachtungsrichtung A. Erkennbar ist, dass die Baugruppe 44 in dieser Ausführungsform um eine weitere Komponente ergänzt wurde, nämlich um eine integrierte Druckanzeigevorrichtung 46, mit der der Druck in dem wenigstens einen Saugkanal 43, 45 angezeigt werden kann. Die Druckanzeigevorrichtung 46 kann z.B. einen Zeiger zum Anzeigen eines Druckwerts oder ein anders gestaltetes Anzeigeelement haben. Die Druckanzeigevorrichtung muss dabei nicht auf eine hochpräzise Druckmessung geeicht sein. Stattdessen ist es ausreichend, wenn durch das Anzeigeelement eine Indikation gegeben wird, in welchem Druckbereich sich der Druck in etwa bewegt.

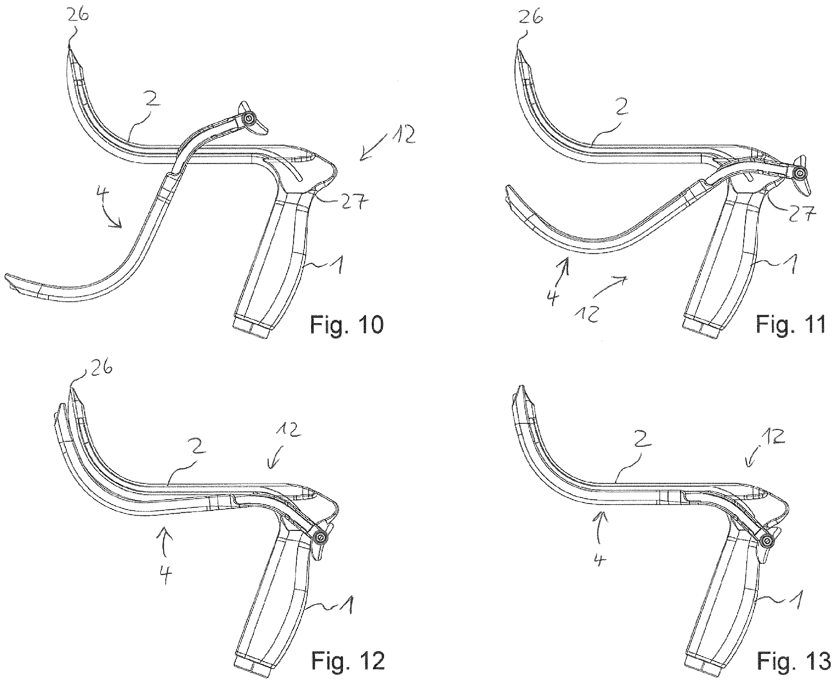

Anhand der Figuren 10 bis 13 wird der Vorgang des Befestigens der Saugvorrichtung 4 am Intubationsgerät 12 beschrieben. Wie die Figur 10 zeigt, wird die Saugvorrichtung 4 zunächst mit der relativ großen Montageöffnung 55 über die Führungsschiene 2 gestülpt, d.h. die Führungsschiene 2 wird durch die Montageöffnung 55 hindurchgeführt. Wie die Figur 11 zeigt, kann auch der Übergangsbereich von der Führungsschiene 2 zum Handgriff 1 durch die Montageöffnung 55 hindurchgeführt werden. Die Saugvorrichtung 4 kann nun, wie die Figur 12 zeigt, so weit verschwenkt werden, dass das patientennahe Befestigungselement 53 an der ersten Befestigungsstelle 26 der Führungsschiene 2 angerastet werden kann und außerdem das patientenferne Befestigungselement 54 an der zweiten Befestigungsstelle 27 zur Anlage gebracht werden kann.

Die Figur 13 zeigt den endgültigen Zustand, in dem die Saugvorrichtung 4 am Intubationsgerät 12 befestigt ist. Das Entfernen der Saugvorrichtung 4 vom Intubationsgerät 12 kann in analoger Weise durchgeführt werden, d.h. in umgekehrter Reihenfolge der Figuren 10 bis 13.

Die erläuterten Ausführungsbeispiele beschreiben eine Saugvorrichtung 4 mit einem relativ starr ausgebildeten Hauptkörper 5. Es ist auch möglich, die Saugvorrichtung bzw. den Hauptkörper in einer elastischen Variante auszubilden, die an und über das Intubationsgerät 12 gespannt wird und dadurch fixiert wird. Während der Unterdruck-Absaugung, d.h. wenn ausreichender Unterdruck in den Saugkanälen vorhanden ist, wird die Stabilität der Anordnung und insbesondere der Saugkanäle weiter erhöht. Die elastische Variante hat den Vorteil, dass der Gesamtdurchmesser der Anordnung nur minimal erhöht wird.

**Sicherheit der Saugaktivität: Kombination** von **Transparenz der Saugvorrichtungnkanäle und Druckanzeige zur Erkennung** von **Fehlfunktionen** Die Transparenz der Saugvorrichtung lässt seitengetrennt klar erkennen, ob die im videolaryngoskopischen Bild zu sehende Flüssigkeit auch tatsächlich effektiv über den linken und/oder rechten Kanal abgesaugt wird - selbst wenn viel Flüssigkeit eine videolaryngoskopische Sicht blockieren sollte.

Wenn allerdings der unwahrscheinliche Fall auftreten sollte, dass sogar beide Ansaugöffnungen 40 Schleimhaut ansaugen bzw. durch Essensreste, Blutkoagel, zähem Sekret, zerfallenem Tumorgewebe o.a. verstopft sind, führt das zu einem Totalausfall der Saugung. Das wird aber unter Umständen überhaupt nicht bemerkt, was besonders bei Auftreten von größeren Flüssigkeitsmengen - wie z.B. bei Regurgitation von Mageninhalt oder ausgeprägten Blutungen nach Tonsillenoperation - zu lebensbedrohlichen Situationen führen kann.

Im klinischen Alltag kann eine verstopfte oder unterbrochene Saugung anhand des verschwindenen, zischenden Geräusches der Vakuumquelle erkannt werden. Das kann aber in Stresssituation überhört werden bzw. unbemerkt bleiben. Zwar gibt es in der Regel eine Druckanzeige direkt an der Vakuumquelle, die ist aber unten am Narkosegerät platziert und daher während der Laryngoskopie meistens nicht einsehbar.

Ein unbemerkter Funktionsausfall kann aber durch eine einfache, gut zu sehende Druckanzeigevorrichtung 46 im Aktivatorbereich am vorderen zum Anwender gewandten Bereich des Handgriffes 1 leicht erkannt werden: Eine Verstopfung lässt sich dann durch einen ausgeprägten Ausschlag in den negativen Druckbereich in Kombination mit der Sicht auf die beiden transparenten Saugkanäle 43, 45 erkennen: Wenn bei einem ausgeprägten Ausschlag in den negativen Druckbereich keine Flüssigkeitsbewegung in den Saugkanälen 43, 45 zu erkennen ist, muss eine Verstopfung beider Ansaugöffnungen 40 mit komplettem Funktionsausfall der Saugung vorliegen.

Bei fehlendem Druckausschlag trotz Aktivierung durch Verschluss der Leckageöffnung 41 mit dem Daumen zeigt die Druckanzeigevorrichtung 46 an der Saugvorrichtung 4 an, ob versehentlich gar kein oder nur ein sehr schwaches Vakuum angeschlossen und/oder eingeschaltet wurde. Auch kann die Sogwirkung durch irgendeine Unterbrechung der Sogleitung bis zur Spitze der Saugvorrichtung reduziert oder sogar aufgehoben sein. So kann die Vakuumquelle Fehlfunktionen aufweisen oder der Schlauch an der Vakuumquelle oder am Saugschienen - Konnektor abgefallen sein. Auch kann die Verbindung zwischen Intubationsgerät-Konnektor und Ansaugöffnung 40 an der Spitze der Saugvorrichtung 4 z.B. durch Materialschäden unterbrochen sein. In diesen Fällen zeigt die Druckanzeigevorrichtung 46 einen fehlenden oder unzureichenden Aufbau eines negativen Druckes an. Die Druckanzeigevorrichtung 46 am Sogaktivator 41 liefert somit eine sichere Kontrolle einer einwandfreien Funktionsfähigkeit der über die Saugvorrichtung erfolgenden Absaugung.

Eine sichere Kontrolle einer einwandfreien Funktionsfähigkeit der Saugung ist beim jetzigen Stand der Technik nur erschwert oder nicht möglich: Die reguläre Druckanzeige an der Vakuumquelle liegt meistens nicht oder nur schlecht während der Laryngoskopie einsehbar im unteren Bereich des Narkosegerätes. Bei einer korrekten Aktivierung des Sogs wird ein Druck von - 500 cmH₂O angezeigt, bei kompletter Obstruktion entsteht ein Unterdruck von - 800 cmH₂O.

Da die Druckanzeige an der Vakuumquelle nicht während der Laryngoskopie eingesehen werden kann, ist ein verminderter oder fehlender Sog in der klinischen Routine nur indirekt zu erkennen: Das zischende Geräusch während der Saugung ist vermindert oder fehlt. Vor allem wird eine Fehlfunktion aber erst dann erkannt, wenn es bereits zu spät ist: Sich ansammelnde Flüssigkeit kann nicht abgesaugt werden und kann dann rasch die Sicht versperren oder bei fehlenden Schutzreflexen in die Lunge gelangen. Besonders bei einer Aspiration von Magensäure kann das wie oben bereits erwähnt zu einer lebensbedrohlichen Lungenschädigung (ARDS) führen.

Wie oben erläutert, kann also eine mechanische oder elektronische Druckanzeigevorrichtung 46 direkt im gut einsehbaren Griffbereich die Sicherheit durch eine verlässliche Kontrolle der Saugleistung erhöhen.

Um z.B. eine Einweg - Saugvorrichtung aufrüsten zu können, kann eine Mehrweg - Druckanzeige z.B. links zwischen Schlauch und Schlauchkonnektor oder als Anzeigering direkt auf der Aktivatoröffnung 41 angebracht werden. Hierbei muss bei Verschluss der Aktivatoröffnung 41 durch den Daumen der Sog bis in den Messbereich im vorderen Teil des Anzeigeringes gelangen können, um hier einen entsprechenden Unterdruck aufbauen und eine verlässliche Druckanzeige hervorrufen zu können.

Da die Absolutwerte nicht von Bedeutung sind, kann diese Anzeige z.B. auch vereinfacht in die drei farblichen Bereiche gelb, orange und rot für "1. nicht aktiv, 2. aktiv, 3. Maximum" eingeteilt werden.

### Akustische Anzeigen einer effektiven Absaugfunktion

Außer oder zusätzlich zu optischen Druckanzeigen können auch akustische Signale anzeigen, ob und in welchem Ausmaß ein effektiver Sog aufgebaut wird: Sicherer als das zur Zeit erzeugte Zischgeräusch könnte z.B. ein zwischen Schlauchansatz und Schlauch angebrachte Vorrichtung einen Pfeifton (bzw. einen angenehmeren Ton) erzeugen: Hierbei ist es wichtig, dass die Saugleistung bei der Erzeugung des Tones nur sehr geringfügig vermindert wird.

Die Tonerzeugung würde allerdings durch das Ansaugen von Flüssigkeit verändert oder verhindert werden. Ein fehlender Ton könnte also sowohl eine fehlende Aktivierung, eine maximale Aktivierung bei korrektem Absaugen von Flüssigkeit, aber auch eine maximale Aktivierung mit Fehlfunktion bei einer Verstopfung beider Absaugöffnungen anzeigen. Die Unterscheidung kann dann aber leicht nach dem oben gezeigten Prinzip erfolgen: Wenn bei aktivierter Saugung im videolaryngoskopischem Bild Flüssigkeit zu sehen ist, kann bei fehlendem Ton anhand der in der transparenten Saugvorrichtung zu erkennenden Flüssigkeit eine korrekte Funktion sicher festgestellt werden. Wenn bei aktiver Saugung und fehlendem Ton allerdings keine Flüssigkeit in der Schiene zu sehen ist, muss eine Verstopfung beider Saugeröffnungen oder eine nicht aktivierte Saugung bzw. eine Unterbrechung der Saugleitung vorliegen.

### Interaktion zwischen Saugvorrichtung und Epiglottislifter

Die Saugvorrichtung 4 ist so konzipiert, dass es keine gegenseitige Beeinflussung bei Einführen, Laryngoskopie, Intubation und Entfernen des Intubationsgeräts 12 und der dabei durchzuführenden Absaugung zwischen Epiglottislifter 3 und Saugvorrichtung 4 gibt. Der Epiglottislifter 3 kann also ohne Behinderungen oder Funktionseinschränkungen durch die Saugvorrichtung 4 auf dem Intubationsgerät 12 positioniert und auch weiterhin frei nach oben und unten bewegt werden.

Das wird durch eine Krümmung der oberen Verbindung zwischen Saugvorrichtung 4 und Aktivatorbereich zum hinteren Teil des Intubationsgerät-Handgriffes 1 erreicht. Hierdurch bleibt der vordere Bereich des Handgriffes 1 frei und der Epiglottislifter 3 kann ungehindert mit seiner dafür vorgesehenen Vorwölbung an der Befestigungskante am oberen Bereich der Führungsschiene 2 hinauf- und auch wieder hinunter geführt werden. Auch kann der Epiglottislifter 3 von der Saugvorrichtung 4 unbeeinflusst vom Intubationsgerät 12 gelöst und dann aus dem Mund entfernt werden.

Umgekehrt wird die Funktion der Saugvorrichtung 4 nicht durch den Epiglottislifter 3 beeinflusst. Die Saugung kann ohne Behinderung durch den Epiglottislifter 3 aktiviert und benutzt werden. Allerdings kann die Saugvorrichtung 4 nur dann an dem Intubationsgerät 12 angebracht und wieder entfernt werden, wenn sich der Epiglottislifter 3 nicht auch an dem Intubationsgerät befindet.

## Patentansprüche

1. Saugvorrichtung (4) eingerichtet zur Anbringung an einem Intubationsgerät (12) zum Intubieren eines Patienten mit einem Endotrachealtubus, wobei das Intubationsgerät (12) einen Handgriff (1) zum Halten des Intubationsgeräts (12) und eine mit dem Handgriff (1) verbundene starre Führungsschiene (2) hat, die einen in Längsrichtung von einem patientenfernen Ende (8) bis zu einem patientennahen Ende (9) der Führungsschiene (2) verlaufenden Führungskanal (23) zum Führen des Endotrachealtubus und wenigstens eine optische Erfassungseinrichtung (11) am patientennahen Ende (9) an der Führungsschiene (2) hat, wobei die Saugvorrichtung (4) zum Absaugen von Flüssigkeiten im Bereich des patientennahen Endes (9) der Führungsschiene (2) eingerichtet ist, **dadurch gekennzeichnet, dass** die Saugvorrichtung (4) wenigstens eine Ansaugöffnung (40) zum Ansaugen der Flüssigkeiten hat, die relativ zu einer beim Intubationsvorgang der Zunge des Patienten zugewandten Oberseite (6) der Führungsschiene (2) an einer Position unterhalb der wenigstens einen optischen Erfassungsvorrichtung (11) oder unterhalb der Führungsschiene (2) angeordnet ist, wenn Saugvorrichtung (4) am Intubationsgerät (12) befestigt ist.

2. Saugvorrichtung nach Anspruch 1, **dadurch gekennzeichnet, dass** die wenigstens eine Ansaugöffnung (40) weiter vom patientenfernen Ende (8) entfernt ist als die wenigstens eine optische Erfassungsvorrichtung (11).

3. Saugvorrichtung nach einem der vorhergehenden Ansprüche, **dadurch gekennzeichnet, dass** die Saugvorrichtung (4) einen Hauptkörper (5) hat, der über seine gesamte Längserstreckung oder zumindest den wesentlichen Teil seiner Längserstreckung einem in Längsrichtung gekrümmten Verlauf der Führungsschiene (2) folgt.

4. Saugvorrichtung nach Anspruch 3, **dadurch gekennzeichnet, dass** der Hauptkörper wenigstens ein patientennahes Befestigungselement (53) hat, mit dem der Hauptkörper (5) an einer ersten Befestigungsstelle (26) am patientennahen Ende (9) der Führungsschiene (2) anrastbar ist, und der Hauptkörper (5) wenigstens ein patientenfernes Befestigungselement (54) hat, mit dem der Hauptkörper (5) an einer zweiten Befestigungsstelle (27) am patientenfernen Ende (8) der Führungsschiene (2) und/oder dem Handgriff (1) befestigbar ist.

5. Saugvorrichtung nach Anspruch 4, **dadurch gekennzeichnet, dass** der Hauptkörper (5) unter Spannung zwischen der ersten und der zweiten Befestigungsstelle (26, 27) einspannbar ist.

6. Saugvorrichtung nach einem der Ansprüche 3 bis 4, **dadurch gekennzeichnet, dass** das patientennahe Befestigungselement (53) als eine in Richtung zum patientenfernen Ende (8) abragende Rastkante ausgebildet ist.

7. Saugvorrichtung nach einem der Ansprüche 3 bis 6, **dadurch gekennzeichnet, dass** der Hauptkörper (5) als im Wesentlichen starres, schienenartiges Bauteil ausgebildet ist.

8. Saugvorrichtung nach einem der Ansprüche 3 bis 7, **dadurch gekennzeichnet, dass** der Hauptkörper (5) einen ersten Seitenabschnitt (51) einen davon beanstandeten zweiten Seitenabschnitt (52) hat, wobei der erste und der zweite Seitenabschnitt (51, 52) im Wesentlichen parallel zueinander in Längsrichtung entlang der Führungsschiene (2) verlaufen, wobei die Führungsschiene (2) zwischen dem ersten und dem zweiten Seitenabschnitt (51, 52) aufnehmbar ist.

9. Saugvorrichtung nach Anspruch 8, **dadurch gekennzeichnet, dass** der erste und der zweite Seitenabschnitt (51, 52) im Bereich des Handgriffs (1) jeweils mit einem Abstand zum Handgriff (1) an diesem vorbeigeführt sind, sodass der erste und der zweite Seitenabschnitt (51, 52) den Handgriff (1) seitlich nicht berühren.

10. Saugvorrichtung nach Anspruch 8 oder 9, **dadurch gekennzeichnet, dass** der erste und der zweite Seitenabschnitt (51, 52) durch einen Bodenabschnitt (50) miteinander verbunden sind.

11. Saugvorrichtung nach Anspruch 10, **dadurch gekennzeichnet, dass** der Bodenabschnitt (50) im am Intubationsgerät (12) befestigten Zustand der Saugvorrichtung (4) unterhalb der Unterseite (7) der Führungsschiene (2) angeordnet ist.

12. Saugvorrichtung nach einem der Ansprüche 3 bis 11, **dadurch gekennzeichnet, dass** der Hauptkörper (5) wenigstens eine Montageöffnung (55) hat, durch die der Handgriff (1) und/oder die Führungsschiene (2) des Intubationsgeräts (12) beim Vorgang des Befestigens der Saugvorrichtung (4) am Intubationsgerät (12) zwischen dem ersten und dem zweiten Seitenabschnitt (51, 52) hindurchführbar ist.

13. Saugvorrichtung nach einem der Ansprüche 3 bis 12, **dadurch gekennzeichnet, dass** der Hauptkörper (5) überwiegend oder vollständig aus farblosem und/oder transparentem Material besteht.

14. Saugvorrichtung nach einem der vorhergehenden Ansprüche, **dadurch gekennzeichnet, dass** die Saugvorrichtung (4) wenigstens einen sich von der wenigstens einen Ansaugöffnung (40) bis zum patientenfernen Ende (8) der Saugvorrichtung (4) erstreckenden Saugkanal (45) hat, der mit einem Anschluss zum Anschließen eines Unterdruckschlauchs verbunden ist.

15. Saugvorrichtung nach einem der vorhergehenden Ansprüche, **dadurch gekennzeichnet, dass** die Saugvorrichtung (4) wenigstens zwei nebeneinander angeordnete, voneinander unabhängige Saugkanäle (43, 45) hat, die jeweils eine Ansaugöffnung (40) am patientennahen Ende (9) der Saugvorrichtung (4) haben und getrennt voneinander sich bis zum patientenfernen Ende (8) der Saugvorrichtung (4) erstrecken.

16. Saugvorrichtung nach einem der Ansprüche 14 bis 15, **dadurch gekennzeichnet, dass** die Saugvorrichtung (4) eine Druckanzeigevorrichtung (46) oder eine Befestigungsaufnahme für eine Druckanzeigevorrichtung (46) hat, wobei durch die Druckanzeigevorrichtung (46) der in dem wenigstens einen Saugkanal (43, 45) vorhandene Druck anzeigbar ist.

17. Saugvorrichtung nach einem der vorhergehenden Ansprüche, **dadurch gekennzeichnet, dass** die Saugvorrichtung (4) eine automatische Fehlererkennung hat, mit der Fehler in der Absaugfunktion der Saugvorrichtung (4) anzeigbar sind.

18. Intubationsgerät (12) zum Intubieren eines Patienten mit einem Endotrachealtubus, mit folgenden Merkmalen:
a) einem Handgriff (1) zum Halten des Intubationsgeräts (12),
b) eine mit dem Handgriff (1) verbundene Führungsschiene (2), die einen in Längsrichtung von einem patientenfernen Ende (8) bis zu einem patientennahen Ende (9) der Führungsschiene (2) verlaufenden Führungskanal (23) zum Führen des Endotrachealtubus und eine beim Intubationsvorgang der Zunge des Patienten zugewandte Oberseite (6) hat,
c) eine Saugvorrichtung (4), die zum Absaugen von Flüssigkeiten im Bereich des patientennahen Endes (9) des Intubationsgeräts (12) eingerichtet ist,
d) **dadurch gekennzeichnet, dass** die Saugvorrichtung (4) wenigstens eine Ansaugöffnung (40) zum Ansaugen der Flüssigkeiten hat, die relativ zu einer beim Intubationsvorgang der Zunge des Patienten zugewandten Oberseite (6) der Führungsschiene (2) an einer Position unterhalb der wenigstens einen optischen Erfassungsvorrichtung (11) oder unterhalb der Führungsschiene (2) angeordnet ist, wenn Saugvorrichtung (4) am Intubationsgerät (12) befestigt ist.
